Europäisches Patentamt

European Patent Office

Office européen des brevets

(11) Numéro de publication: **0 041 458**
**B1**

(12) **FASCICULE DE BREVET EUROPEEN**

(45) Date de publication du fascicule du brevet: **17.04.85**

(51) Int. Cl.⁴: **G 01 N 33/28**

(21) Numéro de dépôt: **81400872.8**

(22) Date de dépôt: **29.05.81**

(54) **Appareil pour la détection du point de trouble d'un liquide constitué d'au moins deux composés ayant des points de congélation différents.**

(30) Priorité: **29.05.80 FR 8011915**

(43) Date de publication de la demande:
**09.12.81 Bulletin 81/49**

(45) Mention de la délivrance du brevet:
**17.04.85 Bulletin 85/16**

(84) Etats contractants désignés:
**DE GB IT**

(56) Documents cités:
**FR-A-1 344 076**
**FR-A-1 357 123**
**FR-A-1 417 250**
**US-A-3 077 764**
**US-A-3 161 039**
**US-A-3 248 928**

**Mc Graw-Hill Encyclopedia of Science and Technology vol. 9 p. 54 (1977)**

(73) Titulaire: **COMPAGNIE FRANCAISE DE RAFFINAGE Société anonyme dite:**
**5, rue Michel-Ange**
**F-75781 Paris Cedex 16 (FR)**

(72) Inventeur: **Solarczyk, Francis**
**99, rue Franchet d'Espérey**
**F-59640 Petite Synthe Dunkerque (FR)**

(74) Mandataire: **Jolly, Jean-Pierre et al**
**Cabinet BROT 83, rue d'Amsterdam**
**F-75008 Paris (FR)**

Courier Press, Leamington Spa, England.

## Description

La présente invention a pour objet un appareil pour la détection du point de trouble d'un liquide, notamment d'un carburant ou d'un combustible.

Certains liquides, d'un usage courant, consistent en des mélanges de composés ayant des points de congélation plus ou moins élevés. Quand on refroidit ces liquides, on constate donc que des cristaux des composés ayant les points de congélation les plus élevés apparaisent au sein du liquide. C'est le cas notamment pour les carburants ou combustibles, connus sous le nom de gazole ou fuel oil, provenant du raffinage du pétrole brut, qui sont constitués par des mélanges d'hydrocarbures. Quand ces carburants ou ces combustibles sont portés à une température suffisamment basse, du fait de la température ambiante par exemple, on constate l'apparition de cristaux constitués par des hydrocarbures aliphatiques saturés. Ces cristaux sont gênants pour l'utilisation d'un gazole alimentant un moteur diesel par exemple, car ils sont susceptibles de perturber le fonctionnement du moteur par col matage des filtres, destinés à retenir d'autres matières solides en suspension dans le gazole, et situés dans le système d'alimentation du moteur.

Ces carburants ou combustibles doivent donc satisfaire à des spécifications fixant la température au-dessus de laquelle ils ne doivent pas contenir de cristaux solides d'hydrocarbures saturés formant un trouble, ou brouillard, dans le produit. Cette température est appelée dans le cas de ces produits, le point de trouble, et sa détermination est effectuée conformément à la norme française NF T 60—105.

Il est entendu que l'appareil constituant la présente invention n'est pas limité à la détermination du point de trouble d'un carburant ou d'un combustible, mais que son application à d'autres liquides présentant les mêmes phénomènes entre dans le cadre de l'invention.

La détermination du point de trouble selon la norme NF T 60—105 nécessite la présence constante d'un opérateur.

En effet, selon cette norme, on abaisse de façon régulière la température de l'échantillon à tester et l'opérateur doit constater de visu la température à laquelle apparaissent les premiers cristaux.

On a donc cherché à mettre au point des appareils permettant de déterminer le point de trouble en réduisant au maximum l'intervention humaine.

Ainsi par FR—A—1.357.123 on connaît un procédé et un appareil pour la détermination du point de trouble d'un liquide constitué d'au moins deux composés ayant des points de congélation différents, selon lesquels:

— on prélève un échantillon dudit liquide,
— on refroidit en continu ledit échantillon,
— on détermine en continu la température dudit échantillon,

— on applique en continu audit échantillon un faisceau lumineux, et
— on détermine le point de trouble par l'intermédiaire de la lumière transmise à travers l'échantillon, et le point de trouble correspondant à la température à laquelle la quantité de lumière détectée atteint une valeur prédéterminée (la lumière transmise diminuant avec l'apparition des cristaux d'hydrocarbures saturés).

Cependant, un tel appareil présente des inconvénients. En effet, le résultat obtenu peut varier d'un échantillon à un autre, même si les deux échantillons présentent en réalité le même point de trouble.

Dans le cas d'un gazole, par exemple:

1) si celui-ci contient de l'eau, des cristaux de glace apparaîtront lors du refroidissement, le point de trouble étant voisin de 0°C, et la quantité de lumière transmise variera avec la quantité d'eau;

2) selon l'origine du pétrole brut à partir duquel est obtenu le gazole, la couleur de celui-ci variera d'un gazole à un autre; la aussi, la quantité de lumière transmise sera différente.

Par le FR—A—1.417.250, on connaît également un procédé et un appareil pour la mesure du point de trouble de liquides, selon lesquels on éclaire, avec de la lumière polarisée, le liquide convenablement refroidi, de manière à réaliser une extinction de lumière en l'absence de cristaux et à déterminer l'augmentation de la lumière traversant le liquide, due à la dépolarisation du faisceau par l'apparition de cristaux anisotropes. De plus, ce document propose de sécher l'échantillon mesuré préalablement à son prélèvement.

Toutefois, du fait que, dans ce procédé, on utilise le faisceau transmis à travers l'échantillon, on retrouve des inconvénients analogues à ceux précédemment mentionnés.

Par ailleurs, on sait, notamment par l'article figurant page 54 du volume 9 de l'encyclopédie "McGraw Hill Encyclopédie of Science and Technologie", que le degré de trouble d'un liquide peut être obtenu par une analyse néphélométrique au moyen d'un appareil dans lequel on mesure la quantité de lumière émise par un échantillon dans une direction perpendiculaire à la direction du faisceau illuminant l'échantillon.

L'invention a plus particulièrement pour but la mise au point d'un appareil donnant des résultats reproductifs lors de la détermination du point de trouble d'un liquide et qui évite les inconvénients précédemment mentionnés.

A cet effet, l'invention a pour objet un appareil pour la mesure automatisée du point de trouble d'un liquide constitué d'au moins deux composés ayant des points de congélation différents, comprenant:

a. une cellule de détermination du point de trouble, équipée:

— d'un émetteur de lumière,
— d'un récepteur de lumière,

— d'un moyen pour repérer la température à l'intérieur de ladite cellule,
— d'un moyen de refroidissement,

b. un dispositif d'alimentation en liquide de ladite cellule permettant de prélever un échantillon dudit liquide et de l'introduire dans ladite cellule,

c. une installation de commande automatique pour contrôler la mesure optique et l'alimentation en liquide,

caractérisé en ce que l'axe optique de recépteur forme un angle sensiblement perpendiculaire avec l'axe optique de l'émetteur, et en ce que le dispositif d'alimentation comprend un premier moyen de séchage consitué par un coalesceur fonctionnant en continu et en outre au moins un deuxième moyen de séchage, ledit second moyen de séchage étant constitué par deux tubes concentriques, le liquide étant introduit dans le tube intérieur dont la paroi laisse passer l'eau, l'intervalle situé entre les deux tubes étant balayé par un gaz ou un liquide anhydre qui entraîne l'eau passant à travers la paroi du tube intérieur, de façon à ce que l'échantillon séché présente moins de 50 p.p.m. d'eau.

On notera que, grâce à l'appareil précédemment décrit, on mesure la quantité de lumière réfléchie par les cristaux qui précipitent les premiers. Parmi ces cristaux, si l'échantillon contient de l'eau, il peut y avoir des cristaux d'eau qui pourraient fausser le résultat, et ne pas donner une valeur représentative du point de trouble. Cependant, conformément au fonctionnement de l'appareil selon l'invention, l'échantillon de liquide testé contient de préférence moins de 50 p.p.m. d'eau grâce à un séchage approprié, et les cristaux d'eau sont donc en faible quantité. Leur présence est donc peu importante pour le résultat.

L'invention est illustrée, de façon non limitative, par les figures 1 à 3 des dessins annexés, qui représentent un appareil, pour la détermination du point de trouble d'un gazole, selon l'invention.

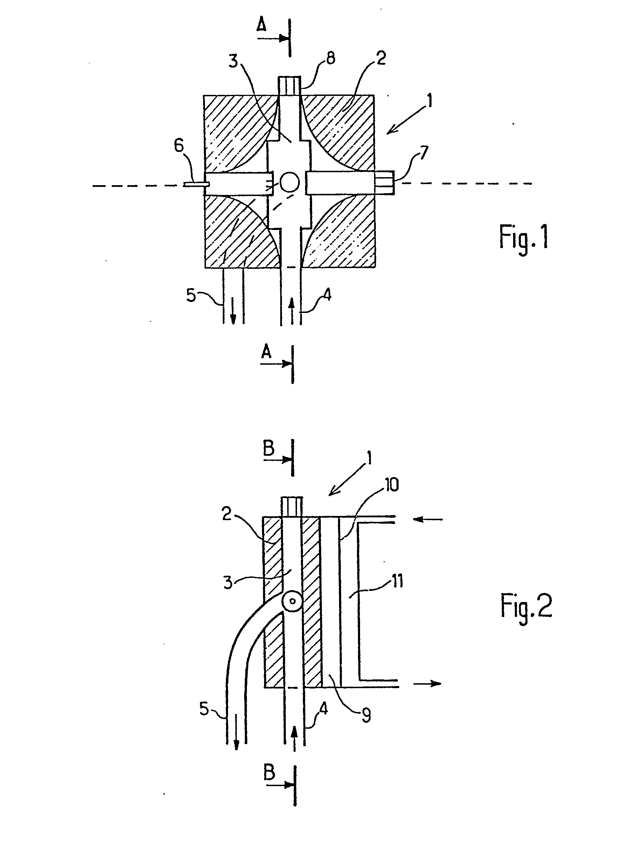

Les figures 1 et 2 représentent la cellule de détermination du point de trouble proprement dit.

La figure 1 est une vue en coupe selon BB de la figure 2.

La figure 2 est une vue en coupe selon AA de la figure 1.

La figure 3 représente un schéma d'ensemble de l'appareil.

En référence aux figures, l'appareil pour la mise en oeuvre du procédé selon l'invention comprend trois organes principaux:

— une cellule de détermination du point de trouble,
— un dispositif d'alimentation de la cellule en gazole d'une teneur en eau inférieure à 50 p.p.m. et équipée à cet effet d'au moins un moyen de séchage du gazole,
— une installation de commande automatique des différentes opérations du procédé.

En référence aux figures 1 et 2 plus particulièrement, la cellule de mesure 1 est constituée d'un corps 2 dans lequel est disposée une chambre 3. Cette chambre 3 est alimentée en gazole par la ligne 4. Le gazole peut être évacué de la cellule par la ligne 5. La chambre 3 est équipée respectivement:

— d'un moyen 6, par exemple une sonde à résistance, permettant de connaître la température à l'intérieur de la cellule,
— d'un émetteur de lumière 7, constitué par exemple par une ampoule fonctionnant sous un courant de 24 Volts et une puissance de 3 Watts ou d'une diode électroluminescente,
— d'un récepteur de lumière 8, constitué par une cellule photoélectrique.

On peut constater d'après la figure 1, que les axes de l'émetteur 7 et du récepteur 8 forment un angle sensiblement droit.

La cellule est en outre équipée d'un système de refroidissement constitué par exemple par un (ou plusieurs) élément(s) à effet Peltier 9. Dans ce cas, la cellule est munie également d'une circulation d'eau 11 pour évacuer les calories fournies par la partie chaude 10 des éléments à effet Peltier.

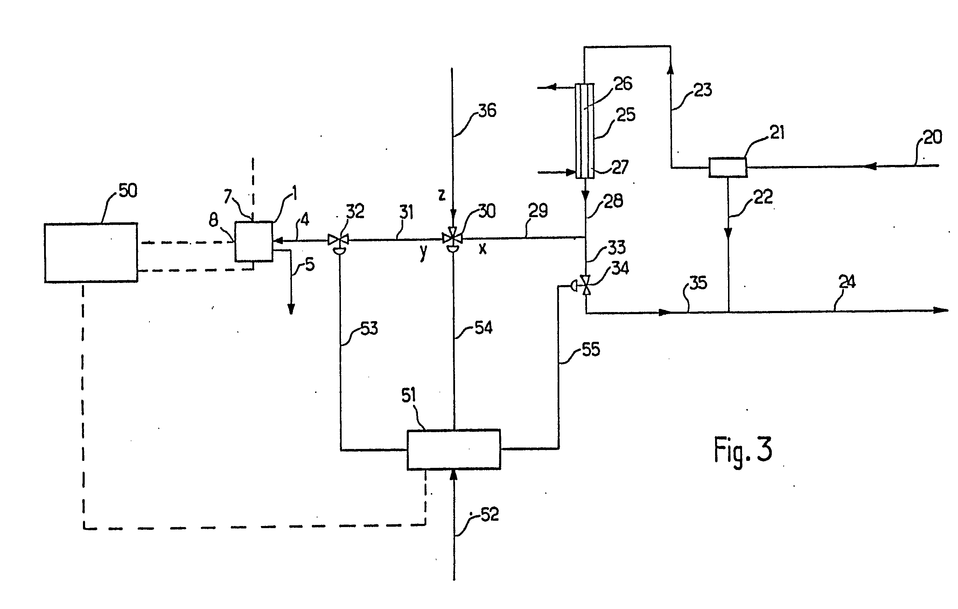

Dans l'exemple représenté figure 3, la cellule est alimentée à partir d'un dispositif d'alimentation permettant de délivrer du gazole présentant une teneur en eau inférieure à 50 p.p.m.

Ce dispositif comprend une ligne 20 d'alimentation en gazole provenant d'une boucle rapide de l'échantillon à analyser pouvant contenir jusqu'à 350 p.p.m. à 500 p.p.m. l'eau.

Le gazole est conduit en continu par la ligne 20 dans un coalesceur 21, où la plus grande partie de l'eau est séparée par coalescence.

On soutire en continu du coalesceur, par la ligne 22, un mélange riche en eau et, par la ligne 23, du gazole pouvant encore contenir jusqu'à 150 p.p.m. d'eau. C'est le gazole contenu dans la ligne 23 qui va servir à fournir l'échantillon sur lequel va être effectuée la détermination du point de trouble du gazole.

Il y a lieu de remarquer que ce coalesceur fonctionne en continu. On peut envisager de le faire fonctionner en discontinu, le soutirage de gazole n'étant effectué que pendant le temps nécessaire au prélèvement d'un échantillon. Dans ce cas cependant, le fonctionnement du coalesceur 21 est perturbé et le gazole contenu dans la ligne 23 peut contenir jusqu'à 350 p.p.m. à 500 p.p.m. d'eau.

Le débit de liquide dans la ligne 23 doit être beaucoup plus faible que celui de la ligne 22 afin d'obtenir un bon fonctionnement du coalesceur. Le rapport

$$\frac{\text{débit dans la ligne 23}}{\text{débit dans la ligne 22}}$$

peut être par exemple voisin de 1/10.

Le liquide contenu dans la ligne 22 est évacué par la ligne 24.

Comme indiqué précédemment, le gazole de la ligne 23 peut contenir jusqu'à 150 p.p.m. d'eau. Ce gazole contient encore trop d'eau pour servir directement à la détermination du point de trouble. Il est préalablement séché grâce à un deuxième moyen de séchage 25. Ce deuxième moyen de séchage peut être notamment constitué comme indiqué sur la figure par un (ou plusieurs) tube(s) intérieur(s) 26, dans lequel circule le gazole à sécher, et dont la paroi laisse passer l'eau, mais pas le gazole.

Le tube intérieur 26 est entouré d'un tube extérieur 27 et dans l'intervalle situé entre les deux tubes circule un fluide tel qu'un gaz sec ou qu'un liquide sec, fluide qui pompe l'eau du gazole à sécher. A la sortie du sécheur 25, le gazole contenu dans la ligne 28 peut servir à la détermination du point de trouble.

A partir de la ligne 28, le gazole séché peut être:

— soit évacué directement par l'intermédiaire de la ligne 33, de la vanne 34, de la ligne 35 et de la ligne 24,
— soit conduit à la cellule 1 par l'intermédiaire de la ligne 29, de la vanne à trois voies 30 (ouverte alors selon XY), de la ligne 31, de la vanne 32 et de la ligne 4.

De l'air sous pression (par exemple 2 bars absolus) peut être admis dans la cellule 1 par l'intermédiaire de la ligne 36, de la vanne à trois voies 30 (alors ouverte selon ZY), de la ligne 31, de la vanne 32 et de la ligne 4.

Outre la cellule de détermination et son dispositif d'alimentation précédemment décrits, l'appareil comprend une installation de commande automatique. Cette installation comprend elle-même une unité centrale 50.

Cette unité reçoit les informations suivantes:

— la température à l'intérieur de la cellule 1, grâce à l'élément thermométrique, et
— le signal de détection du point de trouble du gazole.

L'unité 50 commande un organe pneumatique 51 alimenté en air sous pression (2,5 bars absolus par exemple) par la ligne 52.

Cet organe 51 commande, par l'intermédiaire des lignes 53, 54 et 55 respectivement, les vannes 32, 30 et 34.

L'unité 50 commande également le système de refroidissement de la cellule 1, non représenté dans un but de simplification sur la figure 3.

L'unité 50 peut être équipée d'un enregistreur donnant la température de la cellule ou d'un système de mémorisation du point de trouble.

L'installation fonctionne de la façon suivante:

— au début du cycle de fonctionnement, le dispositif d'alimentation en gazole étant en service, la position des vannes est la suivante:
— la vanne 34 est en position ouverte,
— la vanne 30 est en position ouverte selon XY,
— la vanne 32 est en position fermée.

Le gazole de la ligne 28 est donc évacué par la ligne 24.

L'unité 50 commande, par l'intermédiaire de l'organe 51, l'ouverture de la vanne 32 et la fermeture de la vanne 34.

La cellule 1 se remplit de gazole et au bout d'un temps prédéterminé, l'unité 50 commande la fermeture de la vanne 32 et l'ouverture de la vanne 34 et dispose la vanne 30 selon ZY.

L'unité 50 commande le refroidissement de la cellule. Elle mesure alors en continu la quantité de lumière réfléchie en enregistrant la température de la cellule.

Quand la quantité de lumière réfléchie atteint une valeur critique prédéterminée, on considère que le point de trouble est atteint. Cette valeur critique peut être déterminée en réglant l'unité 50 par rapport à un point de trouble déterminé de façon manuelle.

Les informations relatives au point de trouble peuvent être directement affichées et/ou enregistrées dans les cellules de mémoire de l'unité 50.

Quand le point de trouble est atteint, l'unité 50 commande:

— l'arrêt du fonctionnement de refroidissement,
— l'ouverture de la vanne 32.

De l'air sous pression provenant de la ligne 36 est introduit alors dans la cellule 1. L'air chasse le gazole et les cristaux d'eau ou de paraffine qui se sont déposés dans la cellule.

Au bout d'un temps prédéterminé, la vanne 30 est fermée selon ZY, et ouverte selon XY. La vanne 34 est fermée.

Le gazole pénètre dans la cellule et au bout d'un temps de rinçage déterminé, la vanne 32 est fermée, la vanne 34 ouverte et le cycle recommence, la vanne 30 se dispose selon ZY en ouverture.

La Demanderesse a obtenu de très bons résultats en utilisant le procédé selon l'invention.

Pour un gazole dont le point de trouble est de +2°C, elle n'a constaté qu'un écart moyen de 0,04°C, entre le résultat donné par la méthode manuelle selon la norme AFNOR NF T 60—105, et le résultat donné par le procédé selon l'invention.

**Revendication**

Appareil pour la mesure automatisée du point de trouble d'un liquide constitué d'au moins deux composés ayant des points de congélation différents, comprenant:

a. une cellule (1) de détermination du point de trouble, équipée:

— d'un émetteur de lumière (7),
— d'un récepteur de lumière (8),
— d'un moyen (6) pour repérer la température à l'intérieur de ladite cellule,
— d'un moyen de refroidissement (9, 10, 11),

b. un dispositif (4, 20—35) d'alimentation en liquide de ladite cellule permettant de prélever un échantillon dudit liquide et de l'introduire dans ladite cellule,

c. une installation de commande automatique (50, 51) pour contrôler la mesure optique et l'alimentation en liquide,

caractérisé en ce que l'axe optique du récepteur (8) forme un angle sensiblement perpendiculaire avec l'axe optique de l'émetteur (7), et en ce que le dispositif d'alimentation comprend un premier moyen de séchage constitué par un coalesceur (21) fonctionnant en continu et en outre au moins un deuxième moyen de séchage (25), ledit second moyen de séchage étant constitué par deux tubes concentriques (26, 27), le liquide étant introduit dans le tube intérieur (26) dont la paroi laisse passer l'eau, l'intervalle situé entre les deux tubes étant balayé par un gaz ou un liquide anhydre qui entraîne l'eau passant à travers la paroi du tube intérieur, de façon à ce que l'échantillon séché présente moins de 50 p.p.m. d'eau.

**Patentanspruch**

Gerät zur automatischen Messung des Trübungspunktes einer Flüssigkeit, die aus mindestens zwei Bestandteilen mit verschiedenen Gefrierpunkten besteht, mit

a) einer Zelle (1) zur Bestimmung des Trübungspunktes, welche mit

einer Lichtquelle (7),

einem Lichtempfänger (8),

einem Mittel (6) zur Ermittlung der Temperatur im Inneren der Zelle,

einem Abkühlungsmittel (9, 10, 11)

versehen ist,

b) einer Vorrichtung (4, 20—35) zur Speisung der Zelle mit Flüssigkeit, welche die Entnahme einer Probe der Flüssigkeit und deren Einführung in die Zelle erlaubt,

c) einer automatischen Steuereinrichtung (50, 51) zur Überwachung der optischen Messung und der Speisung mit Flüssigkeit,

dadurch gekennzeichnet, daß die optische Achse des Empfängers (8) mit der optischen Achse der Quelle (7) einen im wesentlichen senkrechten Winkel bildet, und daß die Spiesevorrichtung ein ersten Trocknungsmittel bestehend aus einem kontinuierlich arbeitenden Koalierer (21) und außerdem mindestens ein zweites Trocknungsmittel (25) aufweist, welches aus zwei konzentrischen Rohren (26, 27) besteht, wobei die Flüssigkeit in das innere Rohr (26) eingeführt wird, dessen Wandung wasserdurchlässig ist, und der Zwischenraum zwischen den beiden Rohren mit einem wasserfreien Gas oder einer wasserfreien Flüssigkeit gespült wird, welches bzw. welche das durch die Wandung des inneren Rohres hindurchtretende Wasser mitnimmt, so daß die getrocknete Probe einen Wassergehalt kleiner als 50 ppm aufweist.

**Claim**

Apparatus for the automated measurement of the cloud point of a liquid composed of at least two components having different freezing points, comprising:

a. a cell (1) for determining the cloud point, equipped with:

— a light emitter (7),
— a light receiver (8),
— a means (6) for detecting the temperature inside said cell,
— a cooling means (9, 10, 11),

b. a device (4, 20—35) supplying said cell with liquid for taking a sample of said liquid and introducing it into said cell,

c. an automatic control installation (50, 51) for controlling the optical measurement and liquid supply,

characterized in that the optical axis of the receiver (8) forms a substantially perpendicular angle with the optical axis of the emitter (7) and in that the supply device comprises a first drying means formed from a continuous operation blender (21) and in addition at least a second drying means (25), said second drying means being formed by concentric tubes (26, 27), the liquid being introduced into the inner tube (26) whose wall lets the water pass through, the gap between the two tubes being swept by a gas or anhydrous liquid which carries away the water passing through the wall of the inner tube so that the dried sample has less than 50 p.p.m. of water.

Fig.1

Fig.2

Fig. 3

2